# EUROPEAN PATENT SPECIFICATION

(11) **EP 3 734 292 B1**
(45) Date of publication and mention of the grant of the patent: **28.10.2026**
(21) Application number: 18895731.0
(22) Date of filing: 20.12.2018
(51) Int. Cl.: G01N 35/02, G01N 35/08, G01N 35/04, G01N 35/10, G01N 1/40, G01N 1/34, G01N 35/00, B01L 3/00

(54) **CHANNEL STRUCTURE OF NUCLEIC ACID EXTRACTING CARTRIDGE**
KANALSTRUKTUR EINER NUKLEINSÄUREEXTRAKTIONSKARTUSCHE
STRUCTURE DE CANAL DE CARTOUCHE D'EXTRACTION D'ACIDE NUCLÉIQUE

(30) Priority: 28.12.2017 KR 20170182630
(43) Date of publication of application: 04.11.2020
(73) Proprietor: SD Biosensor Inc., Suwon-si, Gyeonggi-do 16690 (KR)
(72) Inventor: CHO, Young Shik, Yongin-Si Gyeonggi-do 17097 (KR); LEE, Hyo Guen, Suwon-Si Gyeonggi-do 16699 (KR); PARK, Hae Joon, Seongnam-Si Gyeonggi-do 13611 (KR); LEE, Sun Young, Suwon-Si Gyeonggi-do 16693 (KR); LIM, Kwan Hun, Suwon-Si Gyeonggi-do 16690 (KR); KIM, In Ae, Gwangmyeong-Si Gyeonggi-do 14248 (KR); KIM, Jae Young, Suwon-Si Gyeonggi-do 16693 (KR); PARK, Hyo Lim, Suwon-Si Gyeonggi-do 16685 (KR); KIM, Dong Hun, Suwon-Si Gyeonggi-do 16700 (KR)
(74) Representative: FRKelly
(86) International application number: PCT/KR2018/016307
(87) International publication number: WO 2019/132405

(56) References cited:
- EP-A1- 2 842 628
- WO-A1-2016/117726
- WO-A2-2014/008381
- JP-A- 2009 258 118
- JP-A- 2017 125 855
- KR-A- 20070 011 525
- KR-B1- 101 642 434
- KR-B1- 101 734 377
- US-A1- 2015 209 789

## Description

### [Technical Field]

The present invention relates to a flow path structure formed in a cartridge for nucleic acid extraction.

### [Background Art]

In modern times, it has become possible to interpret the cause of disease at the gene level with the development of biotechnology. As a result, the demand for manipulation and biochemical analysis of biological specimens to treat or prevent human diseases is increasing.

Also, in addition to the diagnosis of disease, the technology for extracting and analyzing nucleic acids from samples containing cells or biological specimens is required in various fields such as new drug development, preliminary examination of virus or bacterial infection, and forensic science.

Traditional nucleic acid extractors require each device for each processing process (concentration, purification) and require a long time to move to another device after one processing process is completed.

In the prior art (US 6,374,684), in order to solve this problem, the piston head rotates in one cartridge, and a plurality of reagents can be sucked and mixed. WO 2016/117726 A1 and US 2015/209789 A1 describe further fluidic cartridges for nucleic acid extraction according the the prior art.

However, according to the prior art, a complex flow path structure must be implemented in a small piston head to process a plurality of reagents in one cartridge, so that the manufacturing cost of the cartridge can be increased and there is a problem that the entire cartridge may become unusable if a small error in the piston manufacturing process occurs.

Accordingly, research into a more intuitive and easy-to-implement flow path structure has been conducted to solve this problem.

### [Disclosure]

### [Technical Problem]

An purpose of the present invention is to provide a flow path structure of a cartridge for nucleic acid extraction that makes it easier to suck and discharge samples and reagents.

### [Technical Solution]

In order to achieve the above object, the invention provides a cartridge for nucleic acid extraction as defined in appended claim 1.

According to an embodiment of the present invention, the starting points of the plurality of grooves are arranged at the same distance from the shaft hole.

According to an embodiment of the present invention, the starting points of the plurality of grooves are disposed at the same angle from each other.

According to an embodiment of the present invention, the starting points of the plurality of grooves are disposed 45 degrees apart from other neighboring starting points, respectively. According to an embodiment of the present invention, further provided with an additional groove having a different starting point spaced apart from the shaft hole.

According to an embodiment of the present invention, the end points of five adjacent grooves among the plurality of grooves are arranged at the same distance from the shaft hole.

According to an embodiment of the present invention, the pad covering the surface is a rubber pad.

According to the present invention, the rubber pad is provided with through holes overlapping the start and end points of the grooves.

According to one embodiment of the present invention, the surface includes an outer wall surrounding the rubber pad.

According to one embodiment of the invention, the outer wall is formed to have the same height as the thickness of the rubber pad.

### [Effects of the invention]

According to the flow path structure of the cartridge for nucleic acid extraction according to the present invention, a flow path for sucking and discharging samples and reagents is formed in the cartridge body to distinguish a space in which reagents are mixed and a space for movement. This simplifies the structure of the piston and improves the performance of the cartridge.

### [Description of Drawings]

Figure 1 is a conceptual diagram for explaining the flow path structure of a cartridge for nucleic acid extraction according to an embodiment of the present invention.
Figure 2 is a perspective view of a cartridge for nucleic acid extraction apparatus according to an embodiment of the present invention.
Figure 3 is an exploded view of the cartridge for nucleic acid extraction apparatus according to an embodiment of the present invention.

### [Mode for Invention]

Hereinafter, the present invention will be described in more detail with reference to the drawings. In this specification, the same or similar reference numerals are assigned to the same or similar configurations in different embodiments, and the description is replaced with the first description. As used herein, a singular expression includes a plural expression unless the context clearly indicates otherwise. In addition, the suffixes "module" and "region" for components used in the following description are given or mixed only considering the ease of writing the specification, and do not have meanings or roles that are distinguished from each other.

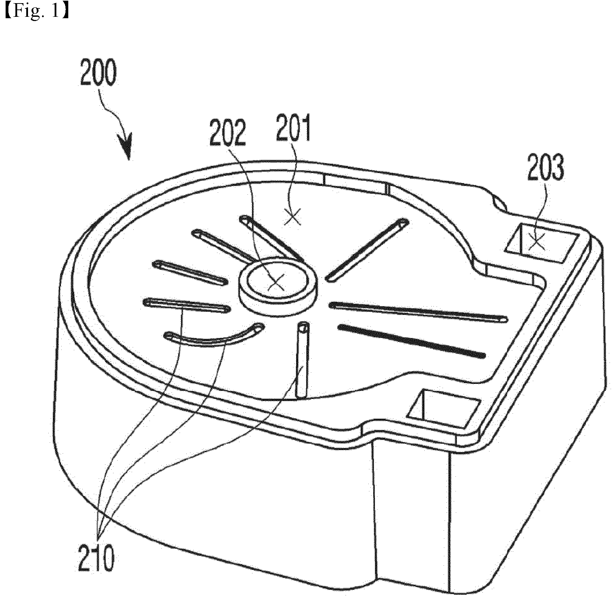

Figure 1 is a conceptual diagram for explaining a flow path structure of a cartridge for nucleic acid extraction according to an embodiment of the present invention.

Referring to FIG. 1, a plurality of grooves (210) extending long may be formed on the surface of the cartridge body (200). The plurality of grooves (210) form a flow path through which samples and reagents move.

A shaft hole (202) may be formed in the center of the body, and a shaft (324) of the piston may be inserted into the shaft hole (202).

The plurality of grooves (210) may be formed to start at the center of the cartridge body (200) and end at the outer region. In other words, the plurality of grooves (210) has a starting point formed around the shaft hole (202) and an end point extending from the starting point toward the outer region of the body..

As shown, the starting points of the plurality of grooves (210) may be arranged at the same distance from the shaft hole (202). That is, the starting points can be placed on a circumference that is the same distance from the center of the shaft hole (202). According to this structure, as the piston rotates, the liquid port formed on the lower body of the piston may overlap the starting point of each flow path.

The starting point of each flow path may be arranged at a certain angle from each other. In other words, the starting points of the plurality of grooves may be arranged at the same angle as the starting points of other grooves adjacent to each other.

For example, according to an embodiment of the present invention, eight flow paths may be formed in the cartridge body, and the starting points of the respective flow path may be arranged at the same angle from each other. That is, the starting point of each flow path may be arranged 45 degrees apart from other neighboring starting points.

According to an embodiment of the present invention, the eight flow paths may be composed of a sample flow path (213), a reagent flow path (211a, 211b, 211c, 211d, 211e, 212), and an amplification module flow path (215).

According to another embodiment of the present invention, an additional flow path (214) may be formed in the cartridge body in addition to the flow paths. The distance from the center of the shaft hole (202) to the starting point of the additional flow path (214) may be formed differently from the distance to the starting point of other flow paths.

When the piston rotates, the liquid port formed on the lower body of the piston overlaps the starting points of the other flow path but does not overlap the starting points of the additional flow path (214). That is, the additional flow path (214) may be used for different purposes from other flow paths. According to an embodiment of the present invention, an additional flow path may be used as a flow path for controlling vacuum when nucleic acids are injected into the amplification module.

As shown, the end points of the five grooves (211a, 211b, 211c, 211d, 211e) constituting the reagent flow path among the plurality of grooves may be disposed on the same circumference.

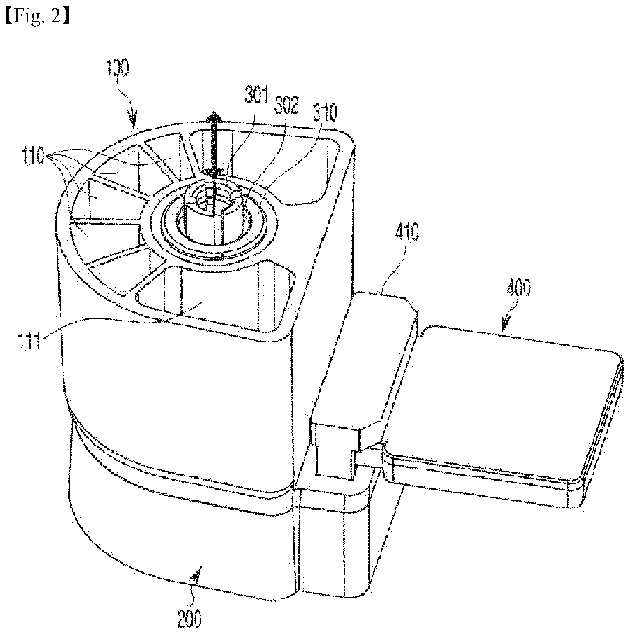

Hereinafter, how the flow path structure described above is implemented in the cartridge will be described in detail with reference to FIGS. 2 to 3.

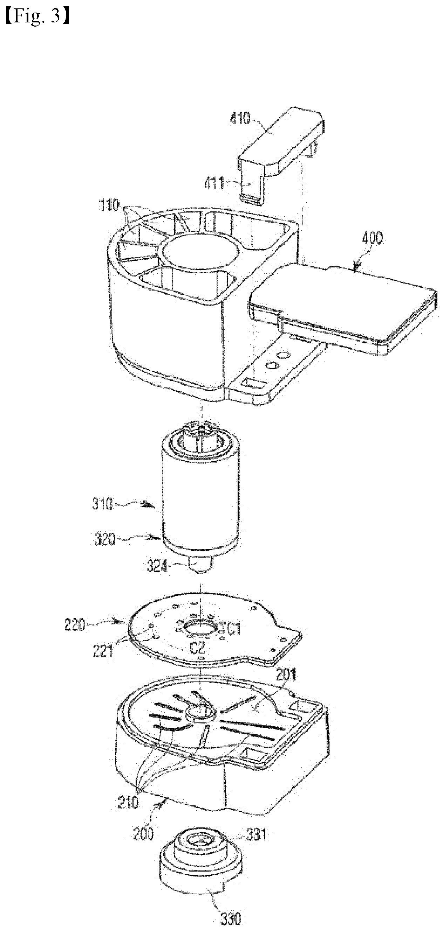

Figure 2 is a perspective view of the cartridge, Figure 3 is an exploded view of the cartridge shown in FIG.2.

Referring to FIG. 2 to 3, the cartridge for nucleic acid extraction may largely be comprised a first body (100), a second body (200), a piston (300), a nucleic acid amplification module (400), and the like.

The first body (100) may be used to store a plurality of reagents.

According to the illustrated, the first body (100) may be formed of a plurality of chambers (110) forming a compartment separated from each other. Different reagents or samples are disposed in each chamber (110) and each chamber (110) forms an independent space so that the reagents do not mix with each other.

The second body (200) guides a path through which the reagent or sample stored in the first body (100) moves.

According to an embodiment of the present invention, the second body (200) may have a liquid flow path through which liquid can move and an air flow path through which air can move, and In order to prevent leakage of the liquid when combined with the first body (100), it may include a pad (220) disposed on the upper surface. When the pad (220) and the cartridge second body (200) are combined, the liquid flow path and the air flow path of the cartridge second body (200) are blocked by the pad (220) to form a space so that the perfect flow path (210) is completed..

The liquid flow path is connected to the rubber pad (220) and the first body (100) to provide a space for samples and reagents to move and mix.

The air flow path connects the amplification module and the vacuum control region of the piston (300) to control the vacuum that can occur when the nucleic acid extracted by the amplification module moves, and serves to prevent contamination of the amplification products that may occur during nucleic acid amplification.

The pad (220) has a plurality of liquid port connection holes, through which the liquid and air flow paths at the bottom of the cartridge are connected to the plurality of reagent chambers (110) located in the cartridge first body (100). The center upper surface of the rubber pad (220) is combined to the bottom surface of the piston lower body (320), and the filter port and the liquid port of the piston lower body (320) rotate while being located in parallel with the liquid port located in the center of the rubber pad (220) and serves to prevent liquid leakage that may occur during liquid movement and mixing.

More specifically, a plurality of flow paths (210) may be formed on the upper region of the second body (200). Each flow path (210) does not cross each other and is formed to extend from the center of the second body (200) to the outer region. As illustrated, some flow paths (210) may have one end disposed on the same circumference and the other end also disposed on the same circumference.

The pad (220) may be combined to the upper region of the second body (200).

The upper region of the second body (200) may be formed with a recessing region (201) recessed toward the bottom, and the pad (220) may be engaged with the recessing region (201) on the upper region of the second body (200).

In other words, an outer wall surrounding the pad may be formed on the upper surface of the second body (200), and the outer wall may be formed to have the same height as the thickness of the pad.

As the pad (220) is in close contact with the upper surface of the second body (200), the flow paths (210) may be sealed. The pad (220) may be formed of rubber or synthetic resin having elasticity so that the pad (220) may be more closely adhered to the second body (200).

A plurality of holes penetrating the pad (220) up and down may be formed in the pad (220).

According to an embodiment of the present invention, the holes are arranged to overlap the top and bottom of ends of the flow paths (210). In other words, holes formed in the pad (220) may be paired in pairs to be connected through the flow path (210).

The pad (220) may comprise a plurality of holes disposed on the same circumference (C1) in the center and a plurality of holes disposed on the same circumference (C2) in the outer region.

The piston (300) may comprise a piston upper body (310) and a piston lower body (320).

In the upper body (310) of the piston (300), an inner space in which reagents and samples can be mixed is formed, and a control rod module of the piston (300) moving up and down may be disposed in the inner space.

The piston control rod module may include a coupling region (301) coupled with a driving region of the nucleic acid extraction device and a sealing region (302) moving up and down in close contact with the piston inner space.

The piston lower body (320) is combined with the piston upper body (310) to form one body.

The piston lower body (320) may be combined with the rotation control module (330).

According to the illustrated, the piston upper body (310) is inserted into the hole formed in the central region of the first body (100) and the shaft (324) of the piston lower body (320) is inserted into the hole formed in the central region of the second body (200).

The shaft (324) of the piston lower body (320) is fixed in engagement with the rotation control module (330) combined to the bottom of the second body (200).

The nucleic acid amplification module (400) may be combined with the first body (100) or the second body (200)
The internal flow path (210) may be formed inside the nucleic acid amplification module (400), and one end of the internal flow path (210) may be formed to overlap with at least one of the flow paths (210) formed in the second body (200).

According to one embodiment of the present invention, there may be a fixing member (410) for covering the nucleic acid amplification module (400) and coupling the first body (100) and the second body (200) so that the nucleic acid amplification module (400) is not arbitrarily separated.

According to the flow path structure of the cartridge for nucleic acid extraction described above, a flow path for sucking and discharging samples and reagents is formed in the cartridge body to distinguish a space in which reagents are mixed and a space for movement. This simplifies the structure of the piston and improves the performance of the cartridge.

The flow path structure of the cartridge for nucleic acid extraction described above is not limited to the configuration and method of the above-described embodiments, but the above embodiments are selectively combined with all or part of each embodiment so that various modifications can be made. The scope of the invention is defined in the appended claims.

## Claims

1. A cartridge for nucleic acid extraction comprising a flow path structure, wherein the cartridge comprises:
a first body (100), which comprises a hole formed in the central region and a plurality of chambers (110);
a second body (200), which is coupled to a lower portion of the first body (100) and comprises a plurality of flow paths (210) and a shaft hole (202) formed in a center of the second body (200);
a piston (300), which comprises a piston upper body (310) that is inserted into the hole formed in the central region and in which an inner space in which reagents and samples can be mixed is formed; wherein the piston (300) comprises a piston lower body (320) which is coupled to the piston upper body (310) and has a port that overlaps with any one of the plurality of flow paths (210) when the piston (300) rotates;
a rotation control module (330), which is coupled to the piston lower body (320);
a piston control rod module, which is configured to move up and down in close contact with the inner space; and
a pad (220), which is disposed on an upper surface of the second body (200), wherein a plurality of holes disposed on a same inner circumference (C1) in the center and a plurality of holes disposed on a same outer circumference (C2) in the outer region are formed through the pad (220),
wherein the plurality of flow paths (210) are formed to extend from the center of the second body (200) to the outer region, and in which the starting point of the plurality of flow paths (210) overlaps with the plurality of holes disposed on the same inner circumference (C1) in the center, and the ending point of the plurality of flow paths (210) overlaps with the plurality of holes disposed on the same outer circumference (C2) in the outer region;wherein when the piston (300) rotates, the starting point of any one flow path of the plurality of flow paths (210) overlaps with the hole on the same inner circumference (C1) in the center and the port of the piston lower body (320), wherein each flow path does not cross each other;
wherein a shaft of the piston lower body (320) is inserted into the shaft hole (202) of the second body (200); and
the piston control rod module includes a coupling region (301) with a driving region of the nucleic acid extraction device and a sealing region (302) moving up and down in close contact with the inner space.

2. The cartridge for nucleic acid extraction according to claim 1,
wherein said starting points of the plurality of flow paths (210) are arranged at the same distance from the shaft hole (202).

3. The cartridge for nucleic acid extraction according to claim 2,
wherein said starting points of the plurality of flow paths (210) are disposed at the same angle from each other.

4. The cartridge for nucleic acid extraction according to claim 2,
wherein the starting points of the plurality of flow paths (210) are disposed 45 degrees apart along the inner circumference (C1).

5. The cartridge for nucleic acid extraction according to claim 4, further comprising an additional flow path (210) having a different starting point spaced apart from the shaft hole.

6. The cartridge for nucleic acid extraction according to claim 4,
wherein said end points of five adjacent flow paths (210) among the plurality of flow paths are arranged at the same distance from the shaft hole (202).

7. The cartridge for nucleic acid extraction according to any one of claims 1 to 6, wherein said pad (220) is a rubber pad.

8. The cartridge for nucleic acid extraction according to claim 7,
wherein said upper surface of the second body (200) comprises an outer wall surrounding the rubber pad.

9. The cartridge for nucleic acid extraction according to claim 8,
wherein the outer wall is formed to have the same height as the thickness of the rubber pad.

## Patentansprüche

1. Kartusche für Nukleinsäureextraktion, umfassend eine Strömungspfadstruktur, wobei die Kartusche Folgendes umfasst:
einen ersten Körper (100), der ein in dem mittigen Bereich ausgebildetes Loch und eine Vielzahl von Kammern (110) umfasst;
einen zweiten Körper (200), der an einen unteren Abschnitt des ersten Körpers (100) gekoppelt ist und eine Vielzahl von Strömungspfaden (210) und ein in einer Mitte des zweiten Körpers (200) ausgebildetes Wellenloch (202) umfasst;
einen Kolben (300), der einen Kolbenoberkörper (310) umfasst, der in das in dem mittigen Bereich ausgebildete Loch eingeführt ist und in dem ein Innenraum, in dem Reagenzien und Proben gemischt werden können, ausgebildet ist; wobei der Kolben (300) einen Kolbenunterkörper (320) umfasst, der an den Kolbenoberkörper (310) gekoppelt ist und eine Öffnung aufweist, die einen beliebigen der Vielzahl von Strömungspfaden (210) überlappt, wenn der Kolben (300) rotiert;
ein Rotationssteuermodul (330), das an den Kolbenunterkörper (320) gekoppelt ist;
ein Kolbensteuerstangenmodul, das konfiguriert ist, sich in engem Kontakt mit dem Innenraum auf und ab zu bewegen; und
ein Polster (220), das auf einer oberen Oberfläche des zweiten Körpers (200) positioniert ist, wobei durch das Polster (220) eine Vielzahl von Löchern, die auf einem selben inneren Umfang (C1) in der Mitte positioniert sind, und eine Vielzahl von Löchern, die auf einem selben äußeren Umfang (C2) in dem äußeren Bereich positioniert sind, ausgebildet sind,
wobei die Vielzahl von Strömungspfaden (210) ausgebildet ist, um sich von der Mitte des zweiten Körpers (200) zu dem äußeren Bereich zu erstrecken, und wobei der Anfangspunkt der Vielzahl von Strömungspfaden (210) die Vielzahl von Löchern überlappt, die auf demselben inneren Umfang (C1) in der Mitte positioniert sind, und der Endpunkt der Vielzahl von Strömungspfaden (210) die Vielzahl von Löchern überlappt, die auf demselben äußeren Umfang (C2) in dem äußeren Bereich positioniert sind; wobei, wenn der Kolben (300) rotiert, der Anfangspunkt eines beliebigen Strömungspfads der Vielzahl von Strömungspfaden (210) das Loch auf demselben inneren Umfang (C1) in der Mitte und die Öffnung des Kolbenunterkörpers (320) überlappt,
wobei keiner der Strömungspfade einander kreuzt;
wobei eine Welle des Kolbenunterkörpers (320) in das Wellenloch (202) des zweiten Körpers (200) eingeführt ist; und
das Kolbensteuerstangenmodul einen Kopplungsbereich (301) mit einem Antriebsbereich der Nukleinsäureextraktionsvorrichtung und einen Dichtungsbereich (302) einschließt, die sich in engem Kontakt mit dem Innenraum auf und ab bewegen.

2. Kartusche für Nukleinsäureextraktion nach Anspruch 1,
wobei die Anfangspunkte der Vielzahl von Strömungspfaden (210) in dem gleichen Abstand von dem Wellenloch (202) angeordnet sind.

3. Kartusche für Nukleinsäureextraktion nach Anspruch 2,
wobei die Anfangspunkte der Vielzahl von Strömungspfaden (210) in dem gleichen Winkel zueinander positioniert sind.

4. Kartusche für Nukleinsäureextraktion nach Anspruch 2,
wobei die Anfangspunkte der Vielzahl von Strömungspfaden (210) 45 Grad voneinander beabstandet entlang des inneren Umfangs (C1) positioniert sind.

5. Kartusche für Nukleinsäureextraktion nach Anspruch 4, ferner umfassend einen zusätzlichen Strömungspfad (210), der einen verschiedenen, von dem Wellenloch beabstandeten, Anfangspunkt aufweist.

6. Kartusche für Nukleinsäureextraktion nach Anspruch 4,
wobei die Endpunkte von fünf benachbarten Strömungspfaden (210) aus der Vielzahl von Strömungspfaden in dem gleichen Abstand von dem Wellenloch (202) angeordnet sind.

7. Kartusche für Nukleinsäureextraktion nach einem der Ansprüche 1 bis 6,
wobei das Polster (220) ein Gummipolster ist.

8. Kartusche für Nukleinsäureextraktion nach Anspruch 7,
wobei die obere Oberfläche des zweiten Körpers (200) eine Außenwand umfasst, die das Gummipolster umgibt.

9. Kartusche für Nukleinsäureextraktion nach Anspruch 8,
wobei die Außenwand ausgebildet ist, um die gleiche Höhe wie die Dicke des Gummipolsters aufzuweisen.

## Revendications

1. Cartouche d'extraction d'acide nucléique comprenant une structure de voies d'écoulement, dans laquelle la cartouche comprend :
un premier corps (100), qui comprend un trou formé dans la région centrale et une pluralité de chambres (110) ;
un second corps (200), qui est couplé à une partie inférieure du premier corps (100) et comprend une pluralité de voies d'écoulement (210) et un trou d'arbre (202) formé au centre du second corps (200) ;
un piston (300), qui comprend un corps supérieur de piston (310) qui est inséré dans le trou formé dans la région centrale et dans lequel un espace intérieur dans lequel des réactifs et des échantillons peuvent être mélangés est formé ;
dans lequel le piston (300) comprend un corps inférieur de piston (320) qui est couplé au corps supérieur de piston (310) et qui a un orifice qui chevauche l'une quelconque de la pluralité de voies d'écoulement (210) lorsque le piston (300) tourne ;
un module de commande de rotation (330), qui est couplé au corps inférieur du piston (320) ;
un module de tige de commande de piston, qui est configuré pour se déplacer de haut en bas en contact étroit avec l'espace intérieur ; et
un coussinet (220), qui est disposé sur une surface supérieure du second corps (200), dans lequel une pluralité de trous disposés sur une même circonférence intérieure (C1) au centre et une pluralité de trous disposés sur une même circonférence extérieure (C2) dans la région extérieure sont formées à travers le coussinet (220),
dans laquelle la pluralité de voies d'écoulement (210) est formée pour s'étendre du centre du second corps (200) vers la région extérieure, et dans laquelle le point de départ de la pluralité de voies d'écoulement (210) chevauche la pluralité de trous disposés sur la même circonférence intérieure (C1) au centre, et le point d'arrivée de la pluralité de voies d'écoulement (210) chevauche la pluralité de trous disposés sur la même circonférence extérieure (C2) dans la région extérieure ; dans laquelle, lorsque le piston (300) tourne, le point de départ de l'une quelconque de la pluralité de voies d'écoulement (210) chevauche le trou situé sur la même circonférence intérieure (C1) au centre et l'orifice du corps inférieur du piston (320),
dans laquelle les voies d'écoulement ne se croisent pas ;
dans laquelle un arbre du corps inférieur du piston (320) est inséré dans le trou d'arbre (202) du second corps (200) ; et
le module de tige de commande du piston comporte une région de couplage (301) avec une région d'entraînement du dispositif d'extraction d'acide nucléique et une région d'étanchéité (302) se déplaçant de haut en bas en contact étroit avec l'espace intérieur.

2. Cartouche d'extraction d'acide nucléique selon la revendication 1,
dans laquelle lesdits points de départ de la pluralité de voies d'écoulement (210) sont disposés à la même distance du trou d'arbre (202).

3. Cartouche d'extraction d'acide nucléique selon la revendication 2,
dans laquelle lesdits points de départ de la pluralité de voies d'écoulement (210) sont disposés au même angle les uns par rapport aux autres.

4. Cartouche d'extraction d'acide nucléique selon la revendication 2,
dans laquelle les points de départ de la pluralité de voies d'écoulement (210) sont disposés à 45 degrés d'intervalle le long de la circonférence intérieure (C1).

5. Cartouche d'extraction d'acide nucléique selon la revendication 4, comprenant en outre une voie d'écoulement supplémentaire (210) ayant un point de départ différent espacé du trou d'arbre.

6. Cartouche d'extraction d'acide nucléique selon la revendication 4,
dans laquelle lesdits points d'extrémité de cinq voies d'écoulement adjacentes (210) parmi la pluralité de voies d'écoulement sont disposés à la même distance du trou d'arbre (202).

7. Cartouche d'extraction d'acide nucléique selon l'une quelconque des revendications 1 à 6,
dans laquelle ledit coussinet (220) est un coussinet en caoutchouc.

8. Cartouche d'extraction d'acide nucléique selon la revendication 7,
dans laquelle ladite surface supérieure du second corps (200) comprend une paroi extérieure entourant le coussinet en caoutchouc.

9. Cartouche d'extraction d'acide nucléique selon la revendication 8,
dans laquelle la paroi extérieure est formée pour avoir la même hauteur que l'épaisseur du coussinet en caoutchouc.
